# EUROPEAN PATENT APPLICATION

(11) **EP 2 174 973 A1**
(43) Date of publication of application: **14.04.2010**
(21) Application number: 08164684.6
(22) Date of filing: 19.09.2008
(51) Int. Cl.: C08G 73/00, A61L 31/00, C08G 67/00, C08G 67/02, C08G 69/00

(54) **Polymeric amines and biomedical uses thereof**

(71) Applicant: Rijksuniversiteit Groningen, 9712 CP Groningen (NL)
(72) Inventor: The designation of the inventor has not yet been filed
(74) Representative: Hatzmann, Martin

(57) **Abstract**

The invention relates to the field of polymers and biomedical applications thereof. In particular, it relates to the use of polymeric amines derived from alternating polyketones. Provided is the use of a polymeric amine for modulating or supporting cellular behavior, said polymeric amine being an alternating aliphatic polyketone comprising N-substituted 2,5-pyrrolediyl groups incorporated in the polymer backbone and bearing an amino functional group pendant from the polymer backbone.

## Description

The invention relates to the field of polymers and biomedical applications thereof. In particular, it relates to the use of polymeric amines derived from alternating polyketones.

Zhang et al. previously disclosed that alternating polyketones can act as excellent precursors for the preparation of polymeric amines (polyamines) by chemical modifications via the Paal-Knorr reaction due to the presence of the highly reactive 1,4-di-carbonyl groups along the backbone.¹ Different kinds of polyamines, containing different amino functionality (which can be either primary, secondary, tertiary or aromatic) as side chains, can be prepared via this kind of modification. It consists just of a two component/one-pot reaction without the need of any catalysts and organic solvent, using mild conditions during the whole process. The easiness of the chemical modifications makes the synthesis of different polyamines (different cross-linking degrees, amount of cationic species, chemical structure of the backbone) a straightforward, fast and cost-effective process. The resulting polyamines were reported to have some interesting physical properties in aqueous solution, including, a good solubility, a surfactant activity and intrinsic photoluminescence.¹

The present inventors surprisingly observed that a polymeric amine derived from alternating polyketones (i.e. polymeric amine being an alternating aliphatic polyketone comprising N-substituted 2,5-pyrrolediyl groups incorporated in the polymer backbone and bearing an amino functional group pendant from the polymer backbone) are particularly suitable for various biomedical applications. It was found that such polyamines display a good biocompatibility with (mammalian) cells when cross-linked to a high degree, whereas polyamines without cross-linking or at low cross-linking levels are useful as apoptosis-inducing agent. The present finding that the properties of these polymers can be fine tuned according to specific needs and applications by the cross-linking degree makes them very attractive and versatile modulators of cell behaviour, such as cell growth, proliferation and apoptosis. The polyamines can be used in combination with biomedical products (for example as a solid coating layer) in order to efficiently prevent inflammatory responses. Since the polymer itself displays drug-like properties, additional coating with conventional (e.g. cytostatic) small molecule drugs is no longer required. By virtue of their pro-apoptotic effects, polyamines derived from alternating polyketones may find their application in the design of drugs or medical implants that require cytostatic properties. Furthermore, as a solid the polyamines can be used as bactericidal.

Accordingly, the invention provides the use of a polymeric amine for modulating or supporting cellular behaviour, said polymeric amine being an alternating aliphatic polyketone comprising N-substituted 2,5-pyrrolediyl groups incorporated in the polymer backbone and bearing an amino functional group pendant from the polymer backbone. The term "pendant from" is meant to indicate that the amino functional group is not part of the polymeric backbone. Such polymeric amines are obtainable by chemical modifications of alternating aliphatic polyketones with a set of functional di-amines via the Paal-Knorr reaction. Thus, unless indicated otherwise, the term "polymeric amine" or "polyamine" as used herein refers to polyamines possessing double functionalities: the N-substituted 2,5-pyrrole-diyl group incorporated in the polymer backbone and a substituent containing an amino functionality, which can be either primary, secondary or tertiary and both aliphatic or aromatic.

In a preferred embodiment, the polymeric amine is of the general formula 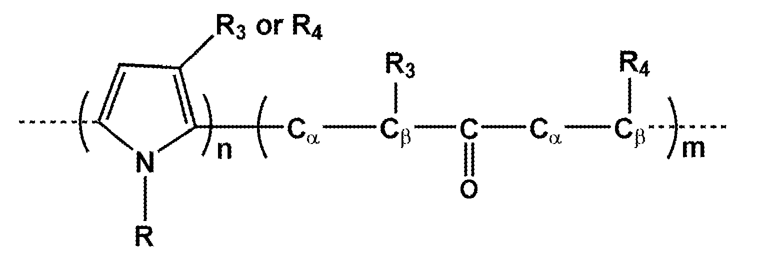 wherein
- the ratio n/m ranges from 0.055 to 4.5;
- R is -X-NR₁R₂
   wherein X is a straight or branched aliphatic chain of 1-10, preferably 1-5 C-atoms, optionally substituted with an amine; and
   wherein R₁, R₂ are independently H or C₁-C₃ alkyl, or wherein R₁ and R₂ together form a ring structure; and
- R₃ and R₄ are independently selected from H, alkyl, aryl, substituted aryl, optionally wherein the alkyl or the aryl substituent contains one or more heteroatoms, like O, N or S.

In one aspect, the amino functionality is a primary amine, i.e. wherein R₁ and R₂ are H.

In a preferred embodiment, R₃ and R₄ are independently selected from H, C₁-C₁₅ aryl and C₁-C₁₅ alkyl groups. More preferably, R₃ and R₄ are independently selected from H, CH₃ and phenyl.

The base polyketone polymers are made by copolymerization of carbon monoxide and non-substituted and substituted olefins. The olefins can be ethylene, propylene or any substituted olefin; the double bond in the olefin can be in the one-position but can also be an internal bond. In addition, the polymerization to obtain the polyketones can take place in a head-to-head and a head-to-tail fashion which implies that the side chains on the polyketone backbone can be present in both the alpha and the beta position as indicated in the structure. This also implies that the substituents R₃ and R₄ present in the 1,4-dicarbonyl unit that reacts with the amine will be present in the pyrrole ring that is formed by the Paal-Knorr reaction; this results in pyrrole rings that have the same substituens R₃ and R₄ in the 3 and/or 4 position on the pyrrole ring.

The molecular weight of the copolymer of carbon monoxide and an olefinically unsaturated compound is not material to the invention and may therefore be selected between wide limits according to the type of application and the method. A low viscosity of the copolymer is advantageous when perfoming chemical modification (Paal-Knorr reaction) with different kinds of diamines in bulk. When the copolymer is a solid, the bulk reaction can be carried out in suspension. Good results have been obtained with a copolymer of carbon monoxide and an olefinically unsaturated compound having a number average molecular weight in the range of from 500 to 100,000, preferably in the range of from 800 to 25,000, more preferably in the range of from 1,000 to 10,000.
Exemplary polyamines include those obtainable by reacting low molecular weight polyketones with a suitable diamine according to the following reaction scheme: 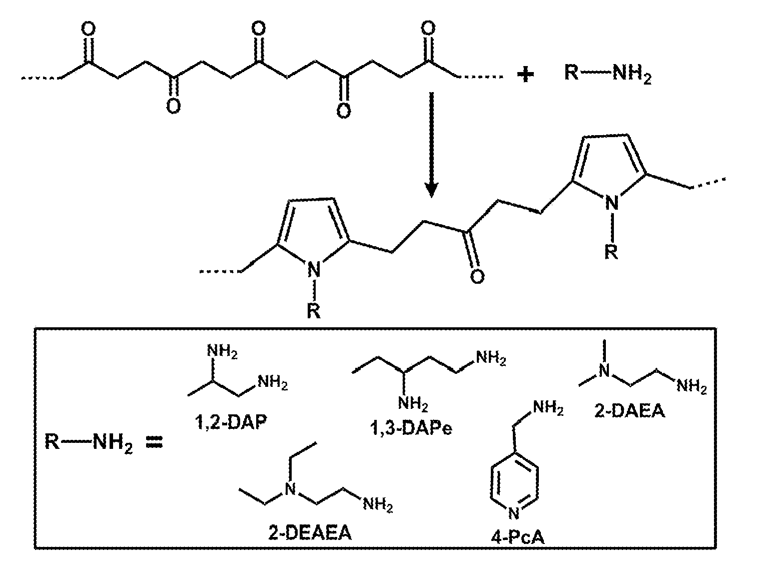 The degree of amino functionality can be finely adjusted by varying the initial molar ratio between the diamines and the 1,4-di-carbonyl functions of the polyketones. The amine addition by the Paal-Knorr reaction needs two carbonyl functions in a 1,4- position to form the pyrrole ring. As the addition of amine to the polyketone backbone is a random process and the conversion level of the carbonyl units can be chosen as desired, the Paal-Knorr reaction delivers a functionalised polymer that has a random distribution of pyrrole rings (in the general formula indicated as n) and residual carbonyl units (indicated as m).

Depending on the desired level of conversion, the carbonyl conversion of the copolymer could range from a low conversion (e.g. 10%) to a high conversion (e.g. 90%) the value for the ratio n/m can run from 0.055 (=10 % conversion) to 4.5 (= 90% conversion). In one aspect, the ratio n/m ranges from about 0.3 to about 1.2. For example, the exemplary polyamines disclosed in the Examples herein below having a conversion of either 40% or 70 % delivers values for n/m of 0.33 and 1.17 respectively.

It was found that a maximum of around 70-90% of carbonyl groups on the polyketone backbone can actually be converted into pyrrole units. Presumably, this is due to both statistical factors (two adjacent carbonyls must react to obtain a ring) and steric hindrance. To exemplify the invention, the examples below describe the properties of polyamines with either a low (2.7 mmol/g) or a high (4.5 mmol/g) degree of amino functionality: one denoted as PA40 having 40% carbonyl conversion and the other denoted PA70 with 70% carbonyl conversion. However, it will be understood that the use of polyamines having higher or lower degrees of amino functionality is also within the scope of the present invention. Typically, the degree of amino functionality ranges from about 2 to about 5 mmol/g. Depending on the type of amino functionality (i.e. either primary, secondary, tertiary or aromatic) and the degree of amino functionality, the polyamines can be cross-linked in a varity of ways, using external cross-linking agents (e.g. glutaraldehyde, dianhydrides, dihalide, etc) or simply just heating at high temperature (preferably above 140 °C). The degree of the cross-linking can be fine tuned by varying the amount of the applied cross-linking agents and the cross-linking time.

As used herein, the expression "modulating or supporting cellular behavior" comprises influencing (either by enhancing, reducing or maintaining) various aspects of cellular homeostasis. Exemplary uses range from enhancing or supporting cell growth or cell proliferation to cytotoxic uses and inducing apoptosis. As is exemplified herein below, the type of the modulating can be optimized according to need or intended purpose by varying the properties of the polyamine, in particular the density of the amino functionality and the degree of cross-linking.

In the past two decades, there has been a growing interest in the use of polymers as biomaterials for application as tissue scaffolds, polymer carriers for drug delivery or medical devices.²⁻⁴ Currently, many studies in tissue engineering are driven by a few classes of polymers such as polylactic acid, polyglycolic acid (PGA), chitosan, polyurethane, and polycaprolactone. These polymers are generally required to be non-toxic, biocompatible with living cells and biodegradable. Polymers containing amino functionality (e.g. polyethylenimine, chitosan, polylysine) have been used and studied for various biomedical applications, such as drug- and DNA-carriers for gene delivery⁵⁻⁶.

In more recent years, a promising class of novel biocompatible materials, alternating polyketones prepared by using homogeneous palladium catalyst systems, have been reported for potential applications in pharmacological and biological fields.⁷⁻¹¹ It is also known in the art that naturally occurring low-molecular aliphatic amines (e.g. putrescine, spermidine and spermine) and structural analogues thereof can induce apoptosis, and can thus be utilized in apoptosis-based cancer therapies.¹²⁻¹⁴ However, the unique and diverse effects of polymeric amines derived from alternating polyketones on different aspects of cell behaviour have not been disclosed or suggested in the art.

One aspect of the invention relates to the use of a polymeric amine for modulating or supporting cellular behaviour comprises modulating or supporting cell growth or proliferation. Provided is the use of polyamine for supporting cell growth or proliferation, wherein said polymeric amine has a medium or high cross-linking density, characterized by a gel content of at least about 90%, preferably at least 95%; and/or a water contact angle of up to 93°, preferably up to 90°.

In one embodiment, the polymeric amine used to support cell growth has an amino functionality of between about 2 and 4 mmol/g and is cross-linked to a medium or high degree into stable bis-pyrrole units. Cross-linking of a polyamine according to the invention is readily achieved by heating, typically to a temperature of at least 140°C, during a suitable period of time. The degree of cross-linking can be determined by elemental analysis. A suitable degree of cross-linking for a polyamine having an amino functionality of between about 2 and 4 mmol (e.g. around 2.7) is for instance reflected by an oxygen content of less than 10%.

As another example of use as a growth supporting polymer, a polymeric amine has an amino functionality of at least 4 mmol/g (e.g around 4.5) and is essentially fully cross-linked to form imine units as determined by an oxygen content of less than 5%.

The cross-linked polyamine may be used in any suitable shape or appearance. For instance, it is a film, a coating or a solid shaped form. In a specific aspect, the polyamine is provided on at least part of the (interior) surface of a conventional culture dish or flask, like a Petri dish. In another embodiment, the polyamine is molded into an implantable device, like a scaffold. Also provided is a polyamine film or molded article being provided with cells, preferably mammalian cells. Also provided is a method for culturing cells, comprising plating or seeding cells onto a solid support comprising cross-linked polyamine.

A further aspect relates to the use of polyamines as cytotoxic or apoptosis-inducing agent. It was found that non-crosslinked polyamines and polyamines crosslinked to a low degree are very suitable to induce the killing of cells, in particular bacterial and mammalian cells. In one embodiment, the invention provides the use of polyamines as anti-bacterial coating. Polyamines having a low cross-linking density may be characterized by a gel content of up to about 96%, preferably up to 90% and/or a water contact angle of at least 90°, preferably at least 93°. In one embodiment, the gel content is less than 85%, like 84, 83, 82 or 81% or even lower. The contact angle of apoptosis-inducing polyamines typically ranges from about 50-55° for non-crosslinked materials up to about 80-85° for low cross-linked materials.

In one embodiment, the polyamine is used as a non cross-linked polymer. For many purposes (e.g. inducing apoptosis *in vitro*) it is convenient to use non-crosslinked polyamine is used in the form of an aqueous solution. It was previously disclosed that polyamines are easily converted into polycations either by the treatment with acid such as lactic acid, citric acid, tartaric acid, acetic acid, toluene sulphonic acid , or by the quaternization of the amino groups (e.g. methylation with methyl bromide or methyl iodide). Therefore, in one embodiment the aqueous solution comprises a dilution of weak acid, for instance acetic acid.

However, polyamines are also advantageously used as cytotoxic or apoptotic materials if they are cross-linked to a low or medium extent. Without wishing to be bound by theory, it is believed that amines released from a solid, crosslinked polyamine, e.g. a film, are responsible for the cell killing effect. In one embodiment, the polymeric amine has an amino functionality of at least 4 mmol/g and is partially cross-linked, for instance reflected by an oxygen content of at least 5%.

Also provided is a shaped object comprising a polyamine, the object being useful for preventing cell growth and/or promoting cell killing. It is for instance a carrier for a cytotoxic drug or a medical (implantable) device. An illustrative example is the use of polyamines for preparing a stent: an implant designed to revascularize obstructed arteries. Current stents are covered with cytostatic drugs to prevent hyperplastic renarrowing of the vessel lumen. A stent comprising or consisting of a polyamine does not require to coating with any further drugs because the polymer material is cytotoxic *per se.*

### LEGENDS TO THE FIGURES

**Figure 1** Cross-link reaction of the polyamines at either a high or a low degree of amino functionality.
**Figure 2** FTIR spectra of polyamines at (A) high (PA70) and (B) low (PA40) degree of amino functionality before and after cross-linking.
**Figure 3** Oxygen content of the polyamines at high (PA70) and low (PA40) degree of amino functionality as a function of cross-linking time.
**Figure 4** Contact angle of the polyamine film at high (PA70) or low (PA40) degree of amino functionality as a function of cross-linking time.
**Figure 5** Apoptosis induction in BAEC by increasing doses of PA70, as evidenced by caspase-3/7 activation. Caspase activity was measured after 24 hours by the capacity of cells to convert a caspase-3/7 substrate into a fluorescent product.
**Figure 6** Morphology of (A) BAEC and (B)VSMC seeded on films prepared from polyamine PA40-PX-h at day 1, day 3, and day 9. (10X magnification)

### EXPERIMENTAL SECTION

**Materials**. The alternating polyketones (Mw 3970), ter-polymers of carbon monoxide, ethylene and propylene were synthesized according to a reported procedure¹⁵. 1,2-diaminopropane (Acros, ≥99%), tetrahydrofuran (THF, Acros, ≥99%) were purchased and used as received.

**Preparation of polymeric amines**. Polymeric amines (polyamines) were synthesized in bulk by reacting two components (polyketones and 1,2-diaminopropane (1,2-DAP)) in a one-pot synthesis. After reaction, the resulting mixtures were washed several times with deionized Milli-Q water. After filtering and freeze-drying, light brown polymers were obtained as the final products. The procedure and characterization of the prepared polyamines are was performed according to Zhang et al. (ref. 1). The polyamines with low (2.7 mmol/g) and high (4.5 mmol/g) degree of amino functionality were prepared here for study, corresponding to 40% and 70% conversion value of carbonyl groups of the polyketones respectively.

**Preparation of polyamine films**. Polyamine films were prepared by a solvent-casting method on glass Petri dishes (Diameter 40 mm). Polyamines were first dissolved in THF at a concentration of 50 mg/ml. After passing through a 200 nm syringe filter twice, 0.5 ml of polyamine solution was added into the Petri dishes, which were then covered with a lid and placed in a fume hood at room temperature overnight for slow evaporation. Cross-linking of the casted films was carried out at 140 °C in vacuum oven at the different time intervals.

**Characterization of polyamine films**. The FTIR spectroscopy was performed using Perkin-Elmer Spectrum 2000. Thermogravimetric analysis (TGA) was conducted in a nitrogen environment on a Perkin-Elmer TGA 7 instrument from 20 °C to 600 °C at a heating rate of 10 °C/min. Elemental analysis of C, H, N were performed with a Euro EA elemental analyzer. The gel content of the cross-linked polyamines was determined by solvent extraction with tetrahydrofuran (THF). Water contact-angle measurements were carried out at room temperature (20 °C) by the sessile drop method, using a custom-built microscope-goniometer system. A 1.5 µl drop of ultrapure water was placed on a freshly prepared sample using a Hamilton micro-syringe and the contact angle was measured after 30 s. At least five different places on the film surface were measured and all quoted angles are subject to an error of ± 2°. The atomic force microscope (AFM) measurements of morphology of polyamine films were performed in tapping mode using a NanoScope IV multimode scanning probe microscope from Digital Instruments. The leaching of polyamines was studied on a HP 8453 Spectrophotometer. A phosphate buffer solution of pH 7.4 was added to the coated Petri dish with polyamines. The coated Petri dish was placed in an oven at 37 °C. After one day, 2 ml was withdrawn from the Petri dish for UV-VIS spectrophotometer analysis.

**Cell culture Studies.** Rat vascular smooth muscle cells (VSMC) and bovine arterial endothelial cells (BAEC) were cultured in Dulbecco's Modified Eagles Medium (DMEM, Life Technologies) supplemented with 10% Fetal calf serum, 2% Penstrep and incubated under 5% CO₂ at 37 °C in a humidified incubator in all of the experiments described herein. The cell morphology was evaluated using a phase contrast, confocal laser microscope at 633 nm wavelength (Zeiss Microsystems LSM, Axiovert 135M) at different time intervals.

**Cell response to polyamine solutions**. The stock solution of polyamines (50 mg/ml) after protonation with acetic acid in de-ionized milli-Q water was diluted with culture medium to form different concentration of polyamines, as indicated in the result section. BAEC were seeded into 96-well plates of polystyrene (10000 cells per well) and incubated for 48 h at 37 °C with a 5 % CO₂ atmosphere. The medium was replaced after 48 h with the medium containing the various concentrations of polyamines. After 24 h, the apoptosis was determined by Caspase-3/7 assay (Promega). The positive control for apoptosis was performed with Menadione (45 µM). The medium was removed and a 50/50 solution of Caspase-3/7 assay and DMEM was added to the cells. The cells were incubated in the dark for 1 h and the fluorescence was recorded on a Wallac Victor 2 1420 multilabel counter luminometer.

**Cell behavior on polyamine films**. Before being seeded with BAEC and VSMC, the polyamine films were first washed with 70% ethanol and three times with culture medium to remove contaminants. The cells were seeded at a density of 800 cells/cm² (i.e. 10000 per dish) for BAEC cells and 1600 cells/cm² for VSMC cells (i.e. 20000 cells per dish). To assess time-dependent cell viability and cell adhesion, the cells were cultured for various durations as indicated in the result section.

### RESULTS

### Characterization of polyamine films

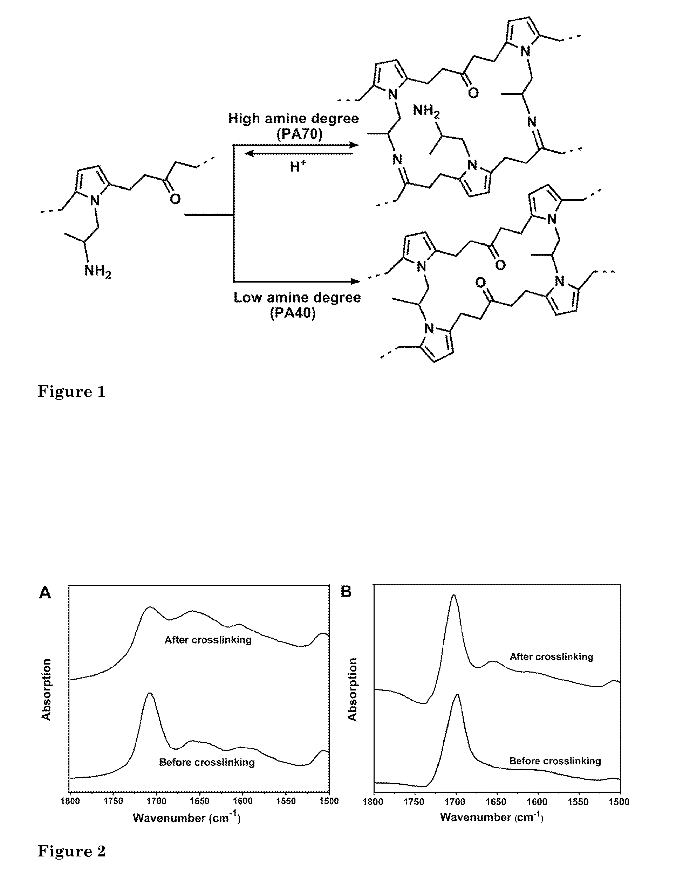

Polymeric amines (polyamines), which have N-substituted 2,5-pyrrolediyl groups incorporated in the backbone bearing an amino functional group pendant from the main chain, were synthesized by reacting a class of low molecular weight polyketones with 1,2-diaminopropane. The route of the chemical modifications of the polyketones described here just consists of a two component/one-pot reaction without the need of any catalysts, organic solvent or any additives. Furthermore, the reaction can be easily carried out by using mild conditions during the whole process. The TGA analysis performed in nitrogen shows a degradation temperature of the polyamines at around 200 °C. The degree of amino functionality was finely adjusted by varying the initial molar ratio between the diamines and the 1,4-di-carbonyl functions of the polyketones. A maximum of around 70-90% of carbonyl groups on the polyketone backbone can be actually converted into pyrrole units. This is due to statistical factors (two adjacent carbonyls must react in order to obtain ring formation) and steric hindrance. Polyamines with low (2.7 mmol/g) and high (4.5 mmol/g) degree of amino functionality were used for study: one with 40% carbonyl conversion (denoted as PA40), and the other with 70% carbonyl conversion (denoted as PA70). Upon heating at high temperature (140 °C), polyamines can be cross-linked (Figure 1) by formation of either instable imine bonds¹⁶ or stable bis-pyrrole units¹⁷. The exact pathway is truly dependent on the degree of amino functionality and in turn on the availability of the 1,4-di-carbonyl functions at the main chain.

FTIR spectra for PA70 and PA40 are presented in Figure 2 before and after cross-linking. The peaks at 1707 cm⁻¹, corresponding to carbonyl group stretching vibration¹⁸, as well as the range of 1500-1680 cm⁻¹ due to the skeletal stretching of the pyrrole ring¹⁹⁻²⁰, represent characteristic absorption for the given system in all the spectra. At high degree of amino functionality (Figure 2A), the intensity of the absorption peak at 1707 cm⁻¹ (carbonyl groups) decreases significantly after cross-linking. This, together with the invariance of the pyrrole rings absorption, constitute an indirect evidence of imine formation. With respect to low degree of amino functionality (Figure 2B), the increased intensity of the absorption peak at 1657 cm⁻¹ (stretching vibration of the pyrrole rings) clearly indicates the bis-pyrrole formation upon cross-linking.

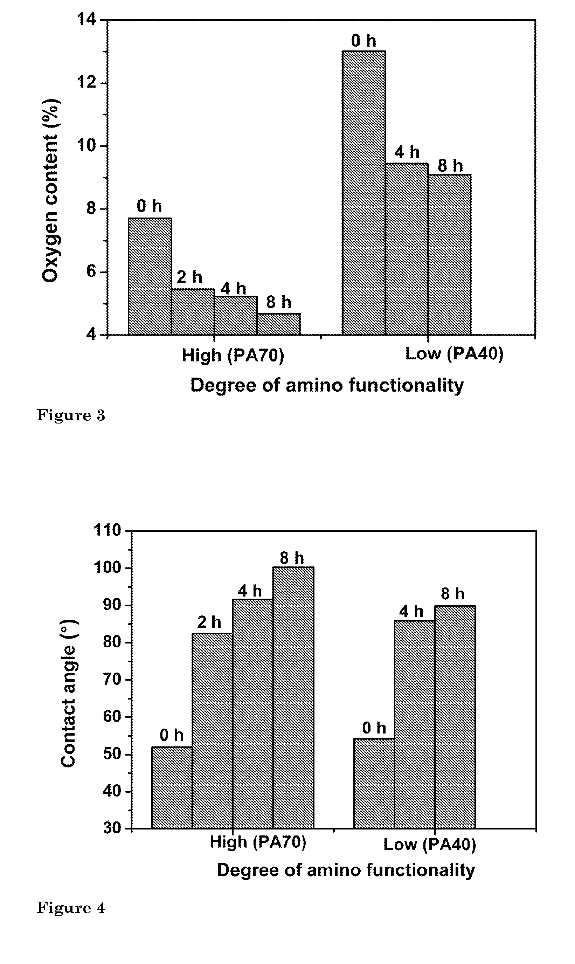

The cross-linking level can be easily tuned as function of the cross-linking time. Such dependence makes it possible to prepare low-cross-linked samples from PA70 after 2 h of cross-linking (PA70-IX-1), medium-cross-linked polyamine after 4 h (PA70-IX-m) and highly-cross-linked polyamine after 8 h (PA70-IX-h). The same holds for PA40 despite the differences in chemical structures of the cross-linked points. For the latter, medium-cross-linked polyamine (PA40-PX-m) and highly-cross-linked polyamine (PA40-PX-h) were prepared after 4 h and 8 h cross-linking time. The increased cross-linking level of the polyamines with the cross-linking time was further verified by the oxygen content values deduced from the elemental analysis (Figure 3). An increase in the cross-linking time leads to a gradual decrease of the oxygen content because of the water formation and release during the cross-linking process. This trend was confirmed by the gel content measurements (Table 1). The gel content of the crosslinked PA70 slightly increases with the cross-linking time and no weight loss is found for the crosslinked PA40 for 4 h and 8 h cross-linking time, indicating a fully-cross-linked structure and stable bispyrrole formation.

**Table 1. Gel content of the polyamines at high (PA70) and low (PA40) degree of amino functionality as a function of cross-linking time.**

| Cross-linked polyamines | Cross-linking Time (h) | Gel content (%) |
|---|---|---|
| PA70-IX-l | 2 | 81 |
| PA70-IX-m | 4 | 90 |
| PA70-IX-h | 8 | 96 |
| PA40-PX-m | 4 | 100 |
| PA40-PX-h | 8 | 100 |

Water contact angle measurements have been commonly used to characterize the relative hydrophilicity or hydrophobicity of polymer surfaces.²¹ The surface of the unmodified polyketones is relatively hydrophilic with an average contact angle of 56°. After chemical modifications, the contact angle of the polyamine film remains virtually unchanged: 52° for PA70 and 54° for PA40 before crosslinking. However, it is interesting to observe that the surface of the polyamines becomes more hydrophobic after cross-linking (Figure 4). The water contact angles of the polyamine surface gradually increase with cross-linking time from 52° to 100° for PA70 and from 54° to 90° for PA40. Such dramatic change can be explained by the fact that the cross-linking reaction between carbonyl groups and amino groups leads to a reduction in the number of the polar groups (amino and carbonyl) at the polymer surface. This also confirms the results of the elemental analysis and gel content with respect to the number of polar groups as a function of cross-linking time. All these observations indicated that the cross-linking process could effectively modify the surface wettability of the polyamines.

Surface morphology of the polyamines before and after cross-linking was examined with atomic force microscope (AFM). The AFM images obtained indicate that the morphology of the surface of PA70 is rather smooth and flat (data not shown). Roughness analysis was performed over the entire image and the surface roughness is expressed as the root mean square (RMS) roughness. The surface roughness of the PA70 thin films before cross-linking is around 0.44 nm, which remains almost unchanged with respect to the cross-linking time: 2 h (0.44 nm), 4 h (0.42 nm) and 8 h (0.52 nm). A very similar surface morphology was observed for the surface of the PA40 thin films before and after cross-linking. Thus, it may be concluded that the crosslinking process has no effect on the surface roughness of the polyamine films.

### Cell culture studies

**Cell response to polyamine solutions.** Since polyamines are easily converted into polycations after treatment with weak acid in water, the effect of a wide concentration range of non-crosslinked polyamine (PA70) after protonation on BAEC was studied in order to determine the cellular response after 4 h by microscopical examination. Depending on the dose of polyamine, substantial changes in cell morphology were detected upon exposure to polyamines (data not shown). Upon treatment with increasing doses of polyamines, BAEC turned into bubbled spheres and partially detached, which is indicative of an apoptotic death.^{22,23} At the highest dose of 250 µg/ml of PA70, cells perished. Cell death was either observed as a loss of cell morphology followed by detachment or an appearance of cytoplasm-free cell remainders at the bottom of the wells, presumably representing bare cytoskeletons. Thus, it seems that at this very high dose necrosis (uncontrolled cell death) becomes the dominant death mechanism.

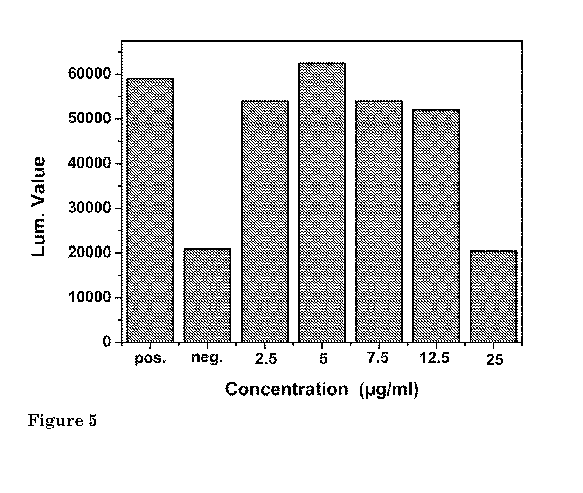

To confirm and further pinpoint the concentration at which apoptosis occurs, activation of the Caspase-3/7 at different doses of polyamine (PA70) was measured (Figure 5). The results show that the cells died from apoptosis already at a concentration of 2.5 µg/ml. At 25 µg/ml of PA70, activation of the Caspase-3/7 was substantially less than those at lower doses (2.5 to 12.5 µg /ml), reaching the level of the negative control experiment. Thus, it may be concluded that at or below doses of 12.5 µg/ml of polyamine, cell death involves apoptosis, while at or above 25 µg/ml of polyamine causes necrosis. The cause of apoptosis in this study may be correlated with the primary amino groups and in turn by the presence of positive charges on the polyamines. However, the exact mechanism of by which polyamines exert their cytotoxicity is unknown.

**Cell behaviour on polyamine films.** It is reported that the use or application of a given biomaterial is closely related to cell behaviour upon contact with them and particularly to cell adhesion to the material solid surface.²⁴ Cell adhesion and proliferation on the polyamine films of PA70 with different cross-linking levels were qualitatively examined using phase contrast microscopy. The cross-linking level of the polyamines was found to play an important role in determining the cell behaviour. The cell behaviour and response to solid films of polyamine (PA70) at different cross-linking level are summarized in Table 2.

**Table 2. Behavior of BAEC and VSMC on the films of PA70-IX-1, PA70-IX-m, and PA70-IX-h at the culture time of 1 h, 1 day, and 4 days. Qualitative scoring from ++ (= high / very well) to - (= absent / poor) is applied.**

| | | PA70-IX-1l | | PA70-IX-m | | PA70-IX-h | |
|---|---|---|---|---|---|---|---|
| Time | Event | BAEC | VSMC | BAEC | VSMC | BAEC | VSMC |
| 1 h | Attachment | + | + | + | + | + | + |
| | Detachment | - | - | - | - | - | - |
| | Cell death | - | - | - | - | - | - |
| 1 day | Attachment | - | +/- | +/- | + | ++ | ++ |
| | Detachment | ++ | +/- | +/- | - | - | - |
| | Cell death | ++ | +/- | +/- | - | - | - |
| 4 days | Attachment | - | - | - | - | ++ | ++ |
| | Detachment | ++ | ++ | ++ | ++ | - | - |
| | Cell death | ++ | ++ | ++ | ++ | - | - |

In general, BAEC and VSMC should attach and stretch out as to maintain viability. Poor attachment and rounding up of these cells are indicative of improper cell-material interaction, which may be followed by cell death due to apoptosis.^{22,23} In 1 h, both BAEC and VSMC started to attach at the surface of PA70 at different cross-linking levels. On day 1, it was observed that BAEC died upon contact with the poorly cross-linked PA70-IX-1. On the medium cross-linked PA70-IX-m, the cells attached yet did not stretch out over the surface. On the highly cross-linked PA70-IX-h, attachment and subsequent morphological change, i.e. stretching out over the surface and developing the typical shape of BAEC, was good (data not shown). With respect to VSMC, a better tolerance to polyamines was observed than for BAEC: some cells were still attached at the film surface and assumed normal VSMC morphology whilst stretching out over the surface on day 1. On day 4, no survived BAEC and VSMC were detected at the surface of PA70-IX-1 and PA70-IX-m. However, PA70-IX-h supports both BAEC and VSMC after 4 days. Both BAEC and VSMC were well-attached and stretched out over the surface of PA70-IX-h to obtain proper morphology. Colonies of VSMC were also observed at the surface of PA70-IX-h on day 4.

The cell behaviour observed might be explained by assuming that polyamine is released from the surface of the partially crosslinked material, and that the released polyamine is responsible for the apoptotic effect. By using UV-Vis spectroscopy, the absorbance at the wavelength of 350 nm, ascribed to pyrrole rings of the polyamines was detected in phosphate buffer at pH of 7.4, indicating the release of the polyamines into cell culture. The release rate of polyamines is considered to depend on the cross-linking level. As a consequence, the cross-linking density (a structural factor) of a polyamine is essential in determining cell survival and a high cross-linking level can lead to the disappearance of the apoptotic effect. The fact that the polyamine induces apoptosis could lead to their potential applications for drug-loaded devices demanding limitation of cell growth.

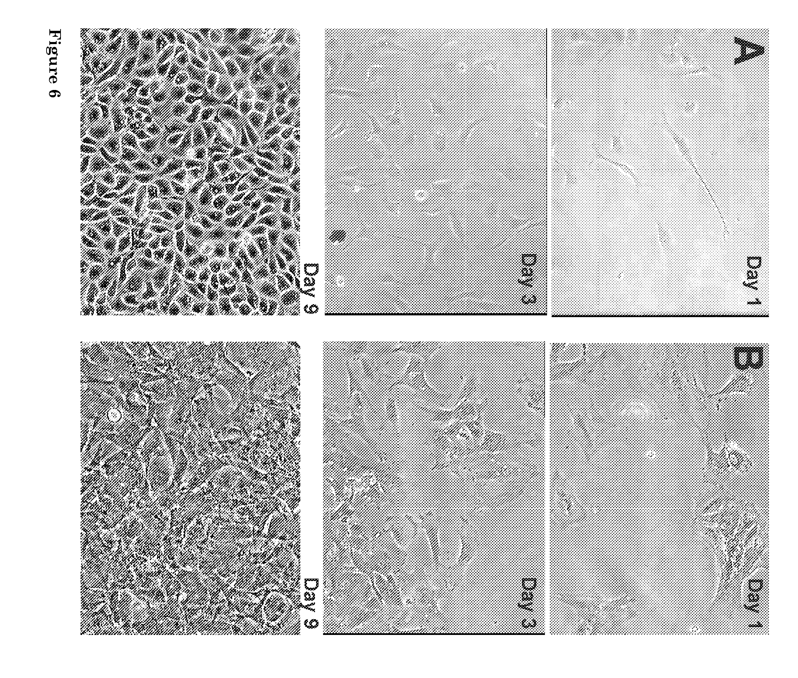

On the other hand, the study of cell behaviour on the films of PA40 after cross-linking demonstrated good biocompatibility with both BAEC and VSMC because of a non-reversible and stable cross-linking bond (bispyrrole). The apoptotic effect completely disappeared for both PA40-PX-m and PA40-PX-h as expected. A similar cell behaviour and morphology were observed for PA40-PX-m and PA40-PX-h. The morphology of BEAC and VSMC on the surface of PA40-PX-h with respect to different culture time is shown as an example (Figure 6). Both types of cells were well-attached at the surface of the PA40-PX-h. The increasing number of both type of cells over time demonstrated the occurrence of the fast proliferation at the surface of polymer films. After 9 days of culture time, both types of cells have grown into confluent layers with a considerably higher cell population density than that of samples on day 3. The morphology of cells grown onto the surface of PA40-PX-h was found to be comparable with that of cells on polystyrene (cells on polyamine films and on polystyrene platforms both reached confluency at day 9). The results for good biocompatibility of PA40 after cross-linking may be utilized to enhance cell adhesion and tissue integration for tissue scaffold application.

### REFERENCES

1. Zhang, Y.; Broekhuis, A. A.; Stuart, M. C. A.; Picchioni, F. J. Appl. Polym. Sci. 2008, 107, 262.
2. Cheung, H.; Lau, K.; Lu, T.; Hui, D. Compos. Pt. B-Eng. 2007, 38, 291.
3. Sokolsky-Papkov, M.; Agashi, K.; Olaye, A; Shakesheff, K.; Domb, A. J. AdvDrug Deliv. Rev. 2007, 59, 187.
4. Jagur-Grodzinski, J. Polym. Adv Technol. 2006, 17, 395.
5. Schmaljohann, D. Adv. Drug Deliv. Rev. 2006, 58, 1655.
6. De-Smedt, S. C.; Demeester, J.; Hennink, W. E. Pharmacol. Res. 2000, 17, 113.
7. Reuter, P.; Fuhrmann, R.; Mucke, A.; Voegele, J.; Rieger, B.; Franke, R. P. Macromol. Biosci. 2003, 3, 123*.*
8. Rohlke, W.; Fuhrmann, R.; Franke, R. P.; Mucke, A.; Voegele, J.; Rieger, B. Macromol. Biosci. 2003, 3, 131.
9. Bartsch, G. C.; Malinova, V.; Volkmer, B. E.; Hautmann, R. E.; Rieger, B, BJU Int. 2006, 99, 447.
10. Malinova, V.; Rieger, B. Macromol. Rapid. Commun. 2005, 26, 945.
11. Malinova, V.; Rieger, B. Biomacromolecules 2006, 7,2931.
12. Wallace, H. M.; Fraser, A. V.; Hughes, A. Biochem. J. 2003, 376, 1.
13. Wallace, H. M.; Fraser A. V. Biochem. Soc. Trans. 2003, 31, 393.
14. Schmitt, C. T.; Lowe, S. W. J. Pathol. 1999, 187, 127.
15. Drent, E.; Keijsper, J. J. US 5225523, 1993.
16. Lu, S.; Paton, R. M.; Green, M. J.; Lucy, A. R. Eur. Polym. J. 1996, 32, 1285.
17. Zhang, Y.; Broekhuis, A. A.; Picchioni, F. J. Appl. Polym. Sci. 2007, 106, 3237*.*
18. Lai, T. W.; Sen, A. Organometallics, 1984, 3, 866.
19. Smela, E.; Zuccarello, G.; Kariis, H.; Liedberg, B. Langmuir 1998, 14, 2970.
20. Mahmud, H. N. M. E.; Kassim, A.; Zainal, Z.; Yunus, W. M. M. J. Appl. Polym. Sci. 2006, 100, 4107.
21. Goddard, J. M.; Hotchkiss, J. H. Prog. Polym. Sci. 2007, 32, 698.
22. Hacker, G. Cell Tissue Res. 2000, 301, 5.
23. Huerta, S.; Goulet, E. J.; Huerta-Yepez, S.; Livingston, E. H. J. Surg. Res. 2007, 139, 143.
24. Anselme, K. Biomaterials 2000, 21, 667.

## Claims

1. Use of a polymeric amine for modulating or supporting cellular behavior, said polymeric amine being an alternating aliphatic polyketone comprising N-substituted 2,5-pyrrolediyl groups incorporated in the polymer backbone and bearing an amino functional group pendant from the polymer backbone.

2. Use according to claim 1, wherein said polymeric amine has the general formula 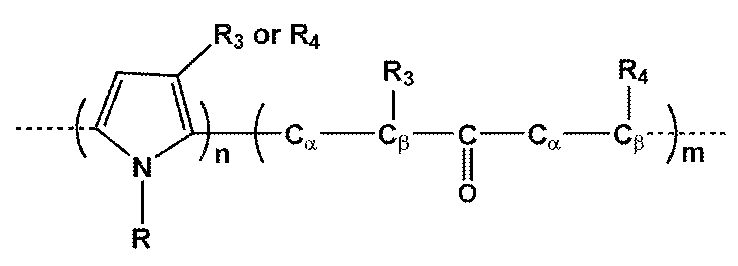 wherein
- the ratio n/m ranges from 0.055 to 4.5;
- R is -X-NR₁R₂
wherein X is a straight or branched aliphatic chain of 1-10, preferably 1-5 C-atoms, optionally substituted with an amine; and
wherein R₁, R₂ are independently H or C₁-C₃ alkyl, or wherein R₁ and R₂ together form a ring structure; and
- R₃ and R₄ are independently selected from H, alkyl, aryl, substituted aryl, optionally wherein the alkyl or the aryl substituent contains one or more heteroatoms, such as O, N or S, preferably wherein R₃ and R₄ are independently selected from H, C₁-C₁₅ aryl and C₁-C₁₅ alkyl groups, more preferably H, CH₃ or phenyl.

3. Use according to claim 2, wherein R₁ and R₂ are H.

4. Use according to claim 2, wherein R is selected from the group consisting of -CH₂-CH(NH₂)-CH₃, -CH₂-CH₂-CH(NH₂)-CH₂-CH₃, -CH₂-CH₂-N(CH₃)₂, -CH₂-CH₂-N(C₂H₅)₂ and -CH₂-C₅H₄N.

5. Use according to any one of the above claims, wherein the degree of amino functionality is between 2 and 5 mmol/g.

6. Use according to any one of claims 1-5 for supporting cell growth or proliferation, wherein said polymeric amine has a medium or high cross-linking density, **characterized by** a gel content of at least about 90%, preferably at least 95%; and/or a water contact angle of up to 93°, preferably up to 90°.

7. Use according to claim 6, wherein said polymeric amine has an amino functionality of between about 2 and 4 mmol/g and which is cross-linked to form stable bis-pyrrole units as determined by an oxygen content of less than 10%.

8. Use according to claim 6, wherein said polymeric amine has an amino functionality of at least 4 mmol/g and which is cross-linked to form imine units as determined by an oxygen content of less than 5%.

9. Use according to any one of claims 6-8, wherein said polymeric amine is in the form of a film, coating or solid shaped form.

10. Use according to any one of claims 1-5 for inducing apoptosis, wherein said polymeric amine has a low cross-linking density, **characterized by** a gel content of up to about 96%, preferably up to 90% and/or a water contact angle of at least 90°, preferably at least 93°.

11. Use according to claim 10, wherein said polyamine is non-crosslinked, preferably wherein said non-crosslinked polyamine is used in the form of an aqueous solution.

12. Use according to claim 11, wherein said polymeric amine has an amino functionality of at least 4 mmol/g and which is partially cross-linked as determined by an oxygen content of at least 5%.

13. Use according to any one of the preceding claims, wherein said polyamine is obtainable by reacting low molecular weight polyketones with a suitable diamine.

14. Scaffold or medical device comprising a polyamine as recited in any one of claims 1-5.

15. A film or molded article comprising a polyamine as recited in any one of claims 1-5, said film or article being provided with cells.

16. A method for culturing cells on a solid support, the support comprising or consisting of a polyamine as recited in any one of claims 1-5.
